# EUROPEAN PATENT SPECIFICATION

(11) **EP 4 504 393 B1**
(45) Date of publication and mention of the grant of the patent: **22.10.2025**
(21) Application number: 23717056.8
(22) Date of filing: 29.03.2023
(51) Int. Cl.: B01J 8/00, B01J 3/00, B01J 4/00, C01C 1/04

(54) **METHOD FOR CONTROLLING A SYNTHESIS LOOP**
VERFAHREN ZUR STEUERUNG EINER SYNTHESESCHLEIFE
PROCÉDÉ DE COMMANDE D'UNE BOUCLE DE SYNTHÈSE

(30) Priority: 06.04.2022 EP 22167015
(43) Date of publication of application: 12.02.2025
(73) Proprietor: CASALE SA, 6900 Lugano (CH)
(72) Inventor: FILIPPI, Ermanno, 6976 Castagnola (CH); PANZA, Sergio, 22100 Como (IT); RIZZI, Maurizio, 22079 Villa Guardia (IT); GALIMBERTI, Leonardo Angelo, 22013 Vercana (CO) (IT)
(74) Representative: M. Zardi & Co S.A.
(86) International application number: PCT/EP2023/058158
(87) International publication number: WO 2023/194178

(56) References cited:
- EP-A1- 3 819 261
- WO-A1-2017/121978
- WO-A1-2021/233780
- GB-A- 2 157 402
- US-A- 2 799 562
- US-B2- 9 463 983
- CCC: "AMMONIA", 1 May 2014 (2014-05-01), pages 1 - 5, XP093129176, Retrieved from the Internet <URL:https://www.cccglobal.com/wp-content/uploads/2016/01/CCC-Brochure-Ammonia.pdf> [retrieved on 20240208]
- STEINKE JOE: "CONTROLLING ANTI-SURGE VALVES IN LOW FLOW LOAD BALANCING OPERATION", 17 INTERNATIONAL CONFERENCE AND EXHIBITION ON LIQUEFIED NATURAL GAS 2013, vol. 3, 28 May 2016 (2016-05-28), pages 1 - 18, XP093038962

## Description

### Field of the invention

The present invention relates to the production of a chemical product, like for example ammonia or methanol, wherein a suitable makeup gas is reacted in a synthesis loop under a loop pressure.

### Prior Art

The industrial production of various chemical products is based on reacting a makeup gas in a so-called synthesis loop. The loop includes at least a synthesis converter, which is usually a catalytic converter, wherein the makeup gas is reacted under suitable conditions of pressure and temperature. The effluent of the converter typically contains unconverted reagents which are separated and recycled to the converter, thus forming the loop. Notable examples are the systems for production of ammonia and methanol.

In most cases of practical interest, the synthesis requires a high pressure. Typically, the synthesis pressure is much higher than the pressure at which the makeup gas is produced. For example in an ammonia synthesis plant, the makeup gas may be produced in a front-end at a pressure around 25-45 bar whereas the loop pressure is well above 100 bar. Therefore the makeup gas is elevated to the loop pressure by a suitable makeup compressor. A second compressor, called circulation compressor, is used to maintain circulation in the loop, i.e. to compensate for the pressure losses, due to the partial conversion of the reactants, in the items and piping of the loop.

The synthesis loop is normally designed to operate with a constant or nearly constant rate of makeup gas feed. This is because the makeup gas is conventionally produced in a front-end running on fossil fuel (e.g. natural gas) which is constantly available and the entire plant is not designed to be flexible, relying on a nearly constant input of makeup gas. Accordingly the above mentioned compressors can be operated at a substantially constant speed of rotation and delivery rate.

In recent years, the need to reduce CO2 emissions and replace fossil fuels with renewable energy sources has emerged. For example, many processes of interest use a hydrogen-containing makeup gas and hydrogen can be produced by electrolysis of water powered with a renewable source, e.g. solar, wind, and others.

However renewable source is intrinsically intermittent and/or variable so that the rate of makeup gas feed, when dependent partially or in full from a renewable energy source, becomes variable.

Controlling a synthesis loop under a highly variable feed rate poses various challenges. For example the flow rate of a compressor may be controlled by varying the speed of rotation, however this is possible only within certain limits to ensure safe operation of the compressor. In a centrifugal compressor, reducing the speed of rotation would also decrease the delivery pressure. In some embodiments, the circulation compressor and the makeup compressor are mounted on the same shaft, so that reducing the speed of the circulation compressor to reduce the flow rate in the loop would also slow down the makeup compressor, lowering the loop pressure at inacceptable level.

If the pressure falls below a certain level, the efficiency of conversion may be severely affected or the reactor may be unable to sustain the reaction of conversion. A significant change in the loop pressure may also affect equilibrium conditions in other equipment of the loop, such as the equilibrium in a condenser. Furthermore, fluctuation of the loop pressure following the fluctuation of the renewable power may rapidly damage the items of the loop, e.g. the pressure vessel of the synthesis reactor, due to fatigue stress.

For the above reasons, the problem of how to control a synthesis loop in the presence of a highly variable feed, due to fluctuation of input power, is not easy to solve.

A control of an ammonia synthesis loop at partial load is disclosed in EP 3 819 261. A method for the control of a loop for preparation of ammonia or methanol is described in WO 2021 233 780.

### Summary of the invention

The invention faces the problem of how to control a synthesis loop for production of a chemical product, particularly for ammonia or methanol, wherein the feed of the loop depends on renewable energy and, consequently, the loop must be controlled to properly follow the fluctuation of said energy.

The problem is solved with a method of claim 1, wherein the pressure of the loop and the delivery rate of said makeup compressor and of said circulation compressor are continuously controlled to follow the variable rate of makeup gas while maintaining the loop pressure within a target deviation from a design loop pressure, wherein said target deviation is no more than 20%. Preferred embodiments are described in the dependent claims.

In the invention, the loop is dynamically controlled to react to the variation of the feed, induced by the fluctuating power of the renewable source, while maintaining the pressure loop close to the design value. The related advantages include a more stable and more reliable operation of the loop; reduced mechanical stress; optimized production based on the available power without the need of a large buffer storage. These advantages will become more evident with the following description.

### Description of the invention

The makeup compressor and the circulation compressor may be reciprocating compressors or centrifugal compressors. Said two types of compressors (make up and circulator) may share the same shaft, so that they rotate at the same speed, or may have separate shafts so that the speed of rotation of each compressor can be controlled independently.

The term compressor denotes the relevant stage(s) of compression. Typically, at least the makeup compressor is multi-stage due to the considerable compression ratio. Each stage of compression may include rotary parts such as an impeller and stationary parts such as a diffuser or stationary vanes. In embodiments with a common shaft, the makeup compressor and the circulation compressor may be regarded as parts of a single machine and may have various structural parts or ancillary equipment in common.

In the invention, the loop pressure is kept within a restricted range of values independently from the loop load by controlling the delivery rate of the compressors through systems like the rotating speed, or by acting on their control systems, like suction valves or other means as described below.

The loop load is measured as the amount of fresh make-up gas fed to the synthesis loop. The loop pressure denotes the operating pressure of the ammonia converter.

### Reciprocating circulation compressor

The circulation compressor may be a reciprocating compressor, i.e. a positive displacement compressor, typically using one or more pistons mounted on a crankshaft.

The delivery rate of a reciprocating circulation compressor is controlled preferably by means of one or more of the following means, including combinations thereof:
a) one or more suction valve unloaders;
b) one or more suction valve modulators;
c) one or more fixed or variable clearance pockets;
d) a bypass system integrated in the compressor;
e) an independent flow throttling valve arranged to control the flow rate in the loop.

A suction valve unloader according to a) is an actuator device arranged to keep, if activated, the compressor suction valve(s) fully open even during the compression phase. The compressor may include a plurality of such suction valve unloaders so that activating them will reduce at steps the delivery rate of the compressor. A suction valve unloader acts on-off on one or more respective suction valves.

A suction valve modulator according to b) is an actuator device having the purpose to modulate the degree of opening of the suction valves during the compression phase. By controlling this device, the delivery rate can be adjusted continuously.

Fixed or variable clearance pockets according to c) are devices arranged to add clearance to the cylinder(s) of a reciprocating compressor, thus varying the quantity of gas actually delivered by each cylinder at each compression phase and, consequently, the delivery rate for a given speed of rotation.

A bypass system according to d) is a system that can recirculate a portion of the flow rate from the discharge side to the suction side.

A flow throttling valve according to e) may be located in principle at any point in the loop, due to the closed configuration of the loop itself. However, a preferred location of said flow throttling valve is at the suction of the circulation compressor and even more preferably immediately upstream the circulator compressor, wherein the term immediately upstream denotes that no equipment (such as a heat exchanger) is installed between the valve and the compressor.

The above systems a) to e) may be adopted singularly or in combination according to different embodiments. Furthermore the above systems a) to d) may be combined with controlling the speed of rotation of the compressor. In case of a reciprocating compressor, the speed of rotation refers to rotation of the crankshaft.

In some embodiments both the makeup compressor and circulation compressor are reciprocating machines. In this embodiment the two said compressors share the same shaft so that they are arranged to rotate always at the same speed. In such a case, the method may comprise controlling the speed of rotation of both compressors by controlling the speed of the common shaft.

In embodiments with reciprocating compressors on the same shaft, controlling the common speed of rotation can adjust the flow rate according to the available feed of makeup gas, whereas it does not affect substantially the loop pressure, which is determined by the compression ratio of the makeup compressor.

### Centrifugal circulation compressor

In certain embodiments the circulation compressor may be a centrifugal compressor, including rotating vaned impellers.

According to preferred embodiments the delivery rate of the circulation compressor, when the compressor is centrifugal, is controlled by one or more of the following means, or combinations thereof:
a) controlling the position of inlet guide vanes of the compressor;
b) controlling the opening of a rate control valve installed at suction or anyway in the loop upstream to the circulator compressor
c) controlling the speed of rotation of the compressor;
Inlet guide vanes according to a) are adjustable stationary vanes at the inlet side of the compressor, which may be arranged to impart a suitable direction or swirl to the inlet flow. By adopting adjustable guide vanes and by controlling their position (also termed degree of opening), the inlet flow can be modified, thus modifying the operation of the compressor and the flow rate that can be discharged by the same.

A control valve according to b) is a valve placed on the suction side of the compressor, which may introduce a variable pressure drop at the inlet of the compressor and, accordingly, may reduce the flow and increase the compression ratio across the machine.

In some embodiments, the makeup gas compressor and the circulation compressor are both centrifugal and mounted on the same shaft, so that they rotate at the same speed. However in a centrifugal compressor, contrary to a reciprocating compressor, the pressure produced at the discharge side depends strongly on the speed of rotation. This means that controlling the speed of the circulation compressor affects the speed of the makeup compressor and, consequently, the pressure loop.

To obviate the above problem, the invention provides, in a preferred embodiment, that:
the speed of rotation of the makeup compressor is controlled to maintain the loop pressure within the target range, the delivery rate of said makeup compressor being controlled separately from the speed of rotation, preferably by a kick-back or bypass system integrated in the compressor;
the delivery rate of the circulation compressor is controlled with any of the means a), b), c) mentioned above, or combinations thereof.

### Preferred embodiments

The following is a description of preferred embodiments based on the type of machine used for the circulator and/or for the makeup gas compressor, i.e. reciprocating or centrifugal, and on the compressors being independent or driven by a common shaft.

In a first embodiment, the circulator is a reciprocating machine, the speed of rotation of said circulator is reduced when the feed decreases and is increased when the feed increases. The reduction or increase of the feed corresponds to reduction or increase of production.

In a second embodiment the circulator is a reciprocating machine and the delivery rate of said circulator is controlled with one or more of the above-mentioned system including suction valve unloader(s); suction valve modulator(s); fixed or variable clearance pockets; integrated bypass system.

In a third embodiment the circulator is a centrifugal machine separated by the makeup compressor and the speed of rotation of said circulator is reduced when the feed decreases and is increased when the feed increases. The speed of the makeup compressor is controlled independently.

In a fourth embodiment the circulator is a centrifugal machine and is controlled by closing a suction valve when the feed decreases and opening said valve when the feed increases, wherein the makeup compressor may be separated or mounted on the same shaft.

In a fifth embodiment the circulator is a centrifugal machine and is controlled by closing inlet guide vanes when the feed decreases and opening said guide vanes when the feed increases, wherein the makeup compressor may be separated or mounted on the same shaft.

In a sixth embodiment the circulator is a reciprocating machine and is controlled by reducing or increasing the speed of rotation according to a reduction or increase of the feed and, at the same time, is controlled by one or more of: suction valve unloader(s); suction valve modulator(s); fixed or variable clearance pockets; integrated bypass system, throttling or opening the independent valve installed at suction or anyway in the loop upstream to the circulator compressor.

In a seventh embodiment the circulator and the makeup gas compressor are separate centrifugal machines and the circulator is controlled by reducing or increasing the speed of rotation according to a reduction or increase of the feed and, at the same time, by controlling the opening of a suction valve and/or the position of adjustable guide vanes at the inlet of the circulator.

In an eighth embodiment the circulator is a centrifugal machine and the makeup gas compressor is a reciprocating machine, and the circulator is controlled by reducing or increasing the speed of rotation according to a reduction or increase of the feed and, at the same time, by controlling the opening of a suction valve and/or the position of adjustable guide vanes at the inlet of the circulator.

In all the above embodiments, the control is performed to maintain the loop pressure within the target range and, preferably, nearly constant or as constant as possible.

### Single compressor machine

In some embodiments, the makeup compressor and the circulator compressor can be realized as centrifugal stages of compression within a single machine. In such embodiments the method of the invention may include a combination of controlling the opening of a suction valve and controlling the position of adjustable guide vanes.

### Further embodiments of the invention

In the various embodiments of the invention, including embodiments with reciprocating or centrifugal compressors, the loop pressure is maintained within a target range. This means, particularly, that the loop pressure is controlled to avoid falling below the design pressure minus the given range in percentage.

In the invention, the pressure of the loop, the delivery rate of the makeup compressor and the delivery rate of the circulation compressor are continuously controlled to follow the variable rate of makeup gas while maintaining the loop pressure within a target deviation from an operating design loop pressure, wherein said target deviation is no more than 20%. A target deviation of no more than 20% may include the case of no variation when the instant pressure is equal to the design pressure of the loop. As the loop pressure may vary with time, the condition above requires that a deviation from loop pressure, if any, is not greater than 20%. For example, assuming the design loop pressure is 100 bar, a target deviation of no more than 20% means the pressure is controlled not to fall below 80 bar.

In preferred embodiments the target range is no more than 15% or no more than 10% or no more than 5%.

In embodiments, said target deviation may have a low limit such as 0.1% or 0.5% or 1.0%. Accordingly, the target deviation may be 0.1% to 20% or 0.5% to 20% or 1.0% to 20%, or ranges with any of the above low limits and an upper limit of any 15%, 10% or 5%.

The invention uses the operating design loop pressure as a setpoint for the method. While the loop pressure is maintained within the target range, the makeup gas feed may vary substantially. In a preferred embodiment, the makeup gas feed can vary within 10% to 100% of a design rate or within 20% to 100% of a design rate, whilst the loop pressure is maintained within the target range. In other words, the method of the invention maintains the loop pressure in the target range for a feed rate which may be as low as 10% of the design feed rate.

In certain embodiments of the invention, a storage of the makeup gas may be provided. Anyway a great advantage of the invention is the ability to follow the rapid fluctuation of the feed rate - dictated by the instant power of the renewable energy source, which means that a storage system, if any, can be small and less expensive. Particularly, a remarkable advantage of the invention is to follow the variable feed rate with substantially no or small variation of the temperature and pressure in the loop, particularly in the synthesis reactor. Hence the reactor does not suffer from pressure waves or heating cycles that may rapidly lead to failure.

The makeup gas in preferred embodiments is a hydrogen-containing gas wherein hydrogen is produced from electrolysis of water, using renewable energy. An interesting application is solar-powered electrolysis of water for the production of hydrogen-containing gas. Among the most interesting application of the invention are the control of an ammonia synthesis loop or a methanol synthesis loop.

An ammonia synthesis loop typically includes: a circulator, a catalytic converter, a condenser, a separator. The converter produces a hot ammonia-containing gaseous product which, after condensation, is separated into a liquid ammonia product and a gaseous phase recycled to the suction of the circulator. The ammonia makeup gas includes hydrogen and nitrogen in a suitable proportion; nitrogen may be obtained e.g. from an air separation unit or PSA.

A methanol synthesis loop is structurally similar to the above-described ammonia synthesis loop. In a methanol synthesis process, the makeup gas is typically a mixture of carbon monoxide and hydrogen.

A particularly preferred embodiment includes generation of hydrogen from electrolysis of water in a suitable electrolyser powered by renewable energy, e.g. solar-powered. It has to be noted that the output of the energy source may vary almost instantaneously, e.g. due to weather conditions. The electrolyser is also able to react in a short time, which is typically a matter of minutes. The loop, according to the invention, is able to react to the variable feed by maintaining a constant or nearly constant pressure, thus avoiding stress of the equipment and maintaining an acceptable efficiency of conversion.

### Description of the figure

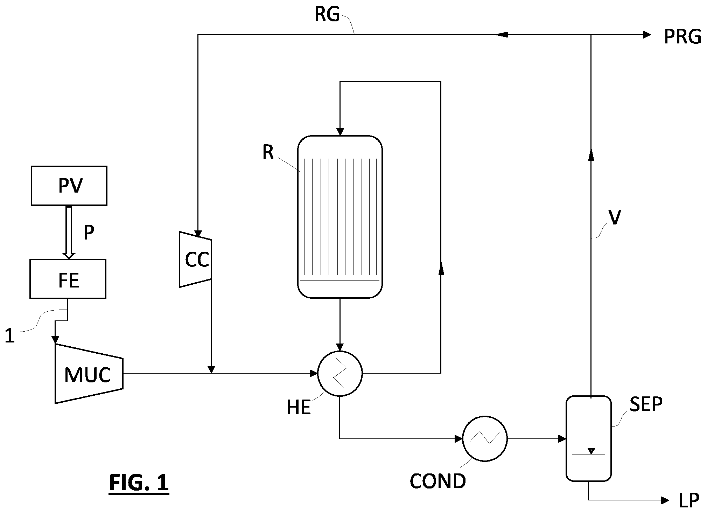

Fig. 1 is a simplified diagram of a loop for the production of a chemical product, for example ammonia or methanol, which can be controlled with the method of the invention.

Fig. 1 illustrates the following main items: makeup gas compressor MUC, circulation compressor CC, reactor R, heat exchanger HE, condenser COND and separator SEP.

A makeup gas 1 is fed to the inlet of the compressor MUC by a front-end FE. The front-end FE is operated with electric power P produced by a renewable source, such as a solar photovoltaic system PV.

The compressed makeup gas is sent to a cold side of the heat exchanger HE, where it is heated with heat removed from the hot effluent of the reactor R, and the heated gas is sent to the reactor R. For example the reactor R is a shell-and-tube equipment where the reaction is carried out in tubes filled with a suitable catalyst and heat of reaction is removed by water or steam in the shell side, or a plate cooled converter where the reaction is carried out in a catalyst bed incorporating cooling plates designed to remove partly or totally the reaction heat.

In other embodiments the reactor R may be a single-bed or multi-bed adiabatic reactor with or without internal heat exchangers. The reactor R may include one or more heat exchangers in the form of tubes or plates.

The effluent of the reactor R, after a passage in the hot side of the exchanger HE, is condensed and liquid fraction LP is separated from a vapour phase V. The liquid fraction LP may represent the liquid product of the loop, e.g. liquid ammonia or liquid methanol. Said vapour phase V is partly recirculated via the circulator CC, a remaining portion PRG is vented to avoid accumulation of inert gas in the loop.

The line RG represents unreacted gas reintroduced in the loop. The discharge of the circulator CC is injected in the delivery line of the main compressor MUC, as shown.

The loop of Fig. 1 reflects a typical loop for the synthesis of ammonia or methanol.

## Claims

1. A method for controlling a synthesis loop for production of a chemical product, wherein:
the loop includes at least a synthesis converter (R), wherein the chemical product is synthesized by reacting a makeup gas (1) containing reagents, and a portion of unconverted reagents (RG) separated from the effluent of the converter (R) is recycled to the converter (R), thus forming said synthesis loop;
the loop operates at a loop pressure, the makeup gas (1) is elevated to said loop pressure by a makeup gas compressor (MUC) and circulation within the loop is maintained by a circulation compressor (CC);
the flow rate of the makeup gas (1) feed depends on at least one source of renewable energy, the feed of makeup gas (1) being time-variable as a consequence, according to the variable power provided by said source;
the pressure of the loop, the delivery rate of said makeup gas compressor (MUC) and the delivery rate of said circulation compressor (CC) are continuously controlled to follow the variable rate of makeup gas (1) while maintaining the loop pressure within a target deviation from an operating design loop pressure, wherein said target deviation is no more than 20%.

2. A method according to claim 1, wherein the circulation compressor (CC) is a reciprocating compressor, i.e. a positive displacement compressor using one or more pistons mounted on a crankshaft.

3. A method according to claim 2, comprising controlling the delivery rate of said circulation compressor (CC) by means of one or more of the following:
a) one or more suction valve unloader(s);
b) one or more suction valve modulator(s);
c) one or more fixed or variable clearance pockets;
d) a bypass system integrated in the compressor;
e) controlling the speed of rotation of the crankshaft;
f) an independent flow throttling valve arranged to control the flow rate in the loop, preferably installed at suction upstream the circulator compressor
g) any combination of the means a) to f).

4. A method according to claim 2 or 3, wherein the makeup gas compressor (MUC) is a reciprocating compressor that share the same shaft of the circulation compressor (CC), so that they are arranged to rotate always at the same speed, and the method comprises controlling the speed of rotation of said compressors.

5. A method according to claims 3 and 4, wherein, specifically, controlling the speed of rotation of the compressors is combined with any of the means a) to d) and f).

6. A method according to claim 1, wherein the circulation compressor (CC) is a centrifugal compressor.

7. A method according to claim 6, comprising controlling the delivery rate of the circulation compressor (CC) by means of one or more of the following:
a) controlling the position of adjustable inlet guide vanes of the compressor;
b) controlling the opening of a rate control valve placed at the suction side of the compressor;
c) controlling the speed of rotation of the compressor;
d) any combination of the means a) to c).

8. A method according to claim 7, wherein the makeup gas compressor (MUC) is a centrifugal compressor on the same shaft of the circulation compressor (CC) so that they have the same speed of rotation.

9. A method according to claim 7, wherein the makeup gas compressor (MUC) is a centrifugal compressor, wherein the speed of rotation of the makeup gas compressor (MUC) and the speed of rotation of the circulation compressor (CC) can be independently controlled, and wherein the method comprises:
the speed of rotation of the makeup gas compressor (MUC) is controlled to maintain the loop pressure within the target range, the delivery rate of said makeup gas compressor (MUC) being controlled separately from the speed of rotation, preferably by a kick-back system integrated in the compressor.

10. A method according to any of the previous claims, wherein said target deviation of the loop pressure is no more than 10%.

11. A method according to the previous claims, wherein the loop pressure is maintained within the target range while the makeup (1) gas feed varies within 10% to 100% of a design rate.

12. A method according to any of the previous claims, wherein the delivery rate of the makeup gas compressor (MUC) and the delivery rate of the circulation compressor (CC) are controlled separately.

13. A method according to any of the previous claims, wherein the makeup gas (1) is a hydrogen-containing gas.

14. A method according to claim 13, wherein hydrogen is at least partially produced by a process of electrolysis of water, which is powered by said renewable energy source.

15. A method according to any of the previous claims, wherein the chemical product is ammonia or wherein the chemical product is methanol.

## Patentansprüche

1. Verfahren zur Steuerung einer Syntheseschleife zur Herstellung eines chemischen Produkts, wobei
die Schleife mindestens einen Synthesekonverter (R) umfasst, wobei das chemische Produkt durch Reaktion eines Zusatzgases (1) enthaltend Reagenzien, synthetisiert wird, und ein Teil von nicht umgewandelten Reagenzien (RG), der vom Abwasser des Konverters (R) getrennt ist, zu dem Konverter (R) recycelt wird, wodurch die Syntheseschleife gebildet wird; der Schleife mit einem Kreislaufdruck arbeitet, das Zusatzgas (1) durch einen Zusatzgasverdichter (MUC) auf diesen Kreislaufdruck erhöht wird und die Zirkulation innerhalb der Schleife durch einen Umlaufverdichter (CC) aufrechterhalten wird;
die Zufuhrmenge des Zusatzgases (1) hängt von mindestens einer erneuerbaren Energiequelle ab, wobei die Zufuhrmenge von Zusatzgas (1) entsprechend der von der Quelle bereitgestellten variablen Leistung zeitlich variabel ist;
der Druck der Schleife, die Fördermenge des Zusatzgasverdichter (MUC) und die Fördermenge des Kreislaufverdichter (CC) werden kontinuierlich so gesteuert, dass sie der variablen Zufuhrmenge des Zusatzgases (1) folgen, während der Kreislaufdruck innerhalb einer Zielabweichung von einem Betriebsauslegungskreislaufdruck gehalten wird, wobei die Zielabweichung nicht mehr als 20 % beträgt.

2. Verfahren nach Anspruch 1, wobei der Kreislaufverdichter (CC) ein Kolbenverdichter ist, d. h. ein Verdrängerverdichter, der einen oder mehrere Kolben verwendet, die auf einer Kurbelwelle montiert sind.

3. Verfahren nach Anspruch 2, eingerichtet zum Steuern der Fördermenge des Kreislaufverdichter (CC) mittels einer oder mehrerer der folgenden Methoden:
ein) ein oder mehrere Saugventilentlader(n);
b) ein oder mehrere Saugventilmodulator(en);
c) eine oder mehrere feste oder variable Entlastungstaschen;
d) ein im Verdichter integriertes Bypass-System;
e) Steuerung der Drehzahl der Kurbelwelle;
f) ein unabhängiges Durchflussdrosselventil, das zur Steuerung der Durchflussmenge in der Schleife angeordnet ist und vorzugsweise an zur Absaugung vor dem Kreislaufverdichter installiert ist;
g) Jede Kombination der Mittel a) bis f).

4. Verfahren nach Anspruch 2 oder 3, wobei der Zusatzgasverdichter (MUC) ein Kolbenverdichter ist, der sich eine Welle mit Kreislaufverdichter (CC) teilt, so dass sie so angeordnet sind, dass sie sich immer mit der gleichen Drehzahl drehen, wobei das Verfahren das Steuern der Drehzahl der Verdichter umfasst.

5. Verfahren nach den Ansprüchen 3 und 4, wobei insbesondere die Steuerung der Drehzahl der Verdichter mit einem der Mittel a) bis d) und f) kombiniert wird.

6. Verfahren nach Anspruch 1, wobei der Umlaufverdichter (CC) ein Zentrifugalverdichter ist.

7. Verfahren nach Anspruch 6, umfassend zum Steuern der Fördermenge des Kreislaufverdichters (CC) mittels einer oder mehrerer der folgenden Methoden:
a) Steuerung der Position der verstellbaren Einlassleitschaufeln des Verdichters;
b) Steuerung der Öffnung eines Mengenregelventils, das sich an der Saugseite des Verdichters befindet;
c) Steuerung der Drehzahl des Verdichters;
d) beliebige Kombination der Mittel a) bis c).

8. Verfahren nach Anspruch 7, wobei der Zusatzgasverdichter (MUC) ein Zentrifugalverdichter auf derselben Welle des Kreislaufverdichters (CC) ist, so dass sie die gleiche Drehzahl aufweisen.

9. Verfahren nach Anspruch 7, wobei der Zusatzgasverdichter (MUC) ein Zentrifugalverdichter ist, wobei die Drehzahl des Zusatzgasverdichters (MUC) und die Drehzahl des Kreislaufverdichters (CC) unabhängig voneinander gesteuert werden können, und wobei das Verfahren umfasst: Steuern der Drehzahl des Zusatzgasverdichter (MUC), um den Kreislaufdruck innerhalb der Zielabweichung zu halten, wobei die Fördermenge des Zusatzgasverdichters (MUC) getrennt von der Drehzahl gesteuert wird, vorzugsweise durch ein in den Verdichter integriertes Rückschlagsystem.

10. Verfahren nach einem der vorhergehenden Ansprüche, wobei die Zielabweichung des Schleifendrucks nicht mehr als 10 % beträgt.

11. Verfahren nach den vorhergehenden Ansprüchen, wobei der Kreislaufdruck innerhalb des Zielbereichs gehalten wird, während die Zuführung von Zusatzgas (1) innerhalb von 10 % bis 100 % einer Auslegungsrate variiert.

12. Verfahren nach einem der vorhergehenden Ansprüche, wobei die Fördermenge des Zusatzgasverdichters (MUC) und die Fördermenge des Kreislaufverdichters (CC) getrennt gesteuert werden.

13. Verfahren nach einem der vorhergehenden Ansprüche, wobei das Zusatzgas (1) ein wasserstoffhaltiges Gas ist.

14. Verfahren nach Anspruch 13, wobei Wasserstoff zumindest teilweise durch einen Prozess der Elektrolyse von Wasser erzeugt wird, welche mit der erneuerbaren Energiequelle betrieben wird.

15. Verfahren nach einem der vorhergehenden Ansprüche, wobei das chemische Produkt Ammoniak ist oder wobei das chemische Produkt Methanol ist.

## Revendications

1. Procédé de commande d'une boucle de synthèse pour la production d'un produit chimique, dans lequel :
la boucle inclut au moins un convertisseur de synthèse (R), dans lequel le produit chimique est synthétisé par réaction d'un gaz d'appoint (1) contenant des réactifs, et une partie de réactifs non convertis (RG) séparée de l'effluent du convertisseur (R) est recyclée vers le convertisseur (R), formant ainsi ladite boucle de synthèse ;
la boucle fonctionne à une pression de boucle, le gaz d'appoint (1) est élevé à ladite pression de boucle par un compresseur de gaz d'appoint (MUC) et la circulation à l'intérieur de la boucle est maintenue par un compresseur de circulation (CC) ;
le débit de l'alimentation en gaz d'appoint (1) dépend d'au moins une source d'énergie renouvelable, l'alimentation en gaz d'appoint (1) étant par conséquent variable dans le temps, selon la puissance variable fournie par ladite source ;
la pression de la boucle, le débit dudit compresseur de gaz d'appoint (MUC) et le débit dudit compresseur de circulation (CC) sont commandés en continu pour suivre le débit variable de gaz d'appoint (1) tout en maintenant la pression de boucle dans une déviation cible par rapport à une pression de boucle de conception de fonctionnement, dans lequel ladite déviation cible n'est pas supérieure à 20 %.

2. Procédé selon la revendication 1, dans lequel le compresseur de circulation (CC) est un compresseur alternatif, c'est-à-dire un compresseur à déplacement positif utilisant un ou plusieurs pistons montés sur un vilebrequin.

3. Procédé selon la revendication 2, comportant la commande du débit dudit compresseur de circulation (CC) au moyen d'un ou plusieurs de ce qui suit :
a) un ou plusieurs dispositif(s) de marche à vide de clapet d'aspiration ;
b) un ou plusieurs modulateur (s) de clapet d'aspiration ;
c) une ou plusieurs poches à dégagement fixe ou variable ;
d) un système de dérivation intégré au compresseur ;
e) la commande de la vitesse de rotation du vilebrequin ;
f) une vanne d'étranglement d'écoulement indépendante agencée pour commander le débit dans la boucle, de préférence installée à l'aspiration en amont du compresseur circulateur
g) toute combinaison des moyens a) à f).

4. Procédé selon la revendication 2 ou 3, dans lequel le compresseur de gaz d'appoint (MUC) est un compresseur alternatif qui partagent le même arbre du compresseur de circulation (CC), de sorte qu'ils sont agencés pour tourner toujours à la même vitesse, et le procédé comporte la commande de la vitesse de rotation desdits compresseurs.

5. Procédé selon les revendications 3 et 4, dans lequel, spécifiquement, la commande de la vitesse de rotation des compresseurs est combinée avec l'un quelconque des moyens a) à d) et f).

6. Procédé selon la revendication 1, dans lequel le compresseur de circulation (CC) est un compresseur centrifuge.

7. Procédé selon la revendication 6, comportant la commande du débit du compresseur de circulation (CC) au moyen d'un ou plusieurs de ce qui suit :
a) la commande de la position des aubes directrices d'admission réglables du compresseur ;
b) la commande de l'ouverture d'une vanne de régulation de débit placée au niveau du côté aspiration du compresseur ;
c) la commande de la vitesse de rotation du compresseur ;
d) toute combinaison des moyens a) à c).

8. Procédé selon la revendication 7, dans lequel le compresseur de gaz d'appoint (MUC) est un compresseur centrifuge sur le même arbre du compresseur de circulation (CC) de sorte qu'ils aient la même vitesse de rotation.

9. Procédé selon la revendication 7, dans lequel le compresseur de gaz d'appoint (MUC) est un compresseur centrifuge, dans lequel la vitesse de rotation du compresseur de gaz d'appoint (MUC) et la vitesse de rotation du compresseur de circulation (CC) peuvent être commandées indépendamment, et dans lequel le procédé comporte :
la vitesse de rotation du compresseur de gaz d'appoint (MUC) est commandée pour maintenir la pression de boucle dans la plage cible, le débit dudit compresseur de gaz d'appoint (MUC) étant commandé séparément de la vitesse de rotation, de préférence par un système de kick-back intégré au compresseur.

10. Procédé selon l'une quelconque des revendications précédentes, dans lequel ledit écart cible de la pression de boucle n'est pas supérieur à 10 %.

11. Procédé selon les revendications précédentes, dans lequel la pression de boucle est maintenue dans la plage cible tandis que l'alimentation en gaz d'appoint (1) varie dans une plage de 10 % à 100 % d'un taux de conception.

12. Procédé selon l'une quelconque des revendications précédentes, dans lequel la vitesse de distribution du compresseur de gaz d'appoint (MUC) et la vitesse de distribution du compresseur de circulation (CC) sont commandées séparément.

13. Procédé selon l'une quelconque des revendications précédentes, dans lequel le gaz d'appoint (1) est un gaz contenant de l'hydrogène.

14. Procédé selon la revendication 13, dans lequel l'hydrogène est au moins partiellement produit par un processus d'électrolyse de l'eau, qui est alimenté par ladite source d'énergie renouvelable.

15. Procédé selon l'une quelconque des revendications précédentes, dans lequel le produit chimique est de l'ammoniac ou dans lequel le produit chimique est du méthanol.
